# EUROPÄISCHE PATENTSCHRIFT

(11) **EP 0 864 579 B1**
(45) Veröffentlichungstag und Bekanntmachung des Hinweises auf die Patenterteilung: **04.06.2003**
(21) Anmeldenummer: 98103904.3
(22) Anmeldetag: 05.03.1998
(51) Int. Cl.: C07H 13/12, A61K 31/70

(54) **Wismutsalze von Antibiotika der Moenomycin-Gruppe, Verfahren zu ihrer Herstellung, ihre Verwendung und solche Salze enthaltende Arzneimittel**
Bismuth salts of moenomycin-like antibiotics, preparation, use and pharmaceuticals containing such salts
Sels de bismuth d'antibiotique, du groupe de la moénomycin, préparation, utilisation, et médicaments les contenant

(30) Priorität: 11.03.1997 DE 19709897
(43) Veröffentlichungstag der Anmeldung: 16.09.1998
(73) Patentinhaber: HOECHST AKTIENGESELLSCHAFT, 65929 Frankfurt am Main (DE)
(72) Erfinder: Vértesy, Lászlo, 65817 Eppstein (DE); Kurz, Michael, Dr., 65719 Hofheim (DE); Markus, Astrid, Dr., 65835 Liederbach (DE); Seibert, Gerhard, Prof. Dr., 64291 Darmstadt (DE)

(56) Entgegenhaltungen:
- EP-A- 0 655 249
- NEW ENGLAND JOURNAL OF MEDICINE, Bd. 333, Nr. 15, 1995, Seiten 984-991, XP000904835
- W.BEIL ET AL.: ARCH. PHARMACOL., Bd. 350, 1994, Seite R1 XP000905015

## Beschreibung

Die vorliegende Erfindung betrifft Wismutsalze von Antibiotika der Moenomycin-Gruppe, Verfahren zu ihrer Herstellung, ihre Verwendung und solche Salze enthaltende Arzneimittel. Die erfindungsgemäßen Salze enthalten einzeln oder im Gemisch vorliegende Antibiotika aus der Moenomycin-Gruppe, das sind sogenannte Phosphoglycolipid-Antibiotika, und das Wismut in definierten stöchiometrischen Verhältnissen. Sie eignen sich in hervorragender Weise insbesondere zur Bekämpfung von Helicobacter pylori und damit zum Beispiel zur Therapie und Prophylaxe von Magenerkrankungen.

Zur Behandlung und Vorbeugung von Magengeschwüren oder der Gastritis sowie zur Prophylaxe von Magenkrebs wurden bisher vor allem sogenannte Antacida und mit besonderem Erfolg H₂-Rezeptorblocker benutzt. Mittlerweile wurde erkannt, daß häufig Infektionen mit dem Mikroorganismus Helicobacter pylori für Magenerkrankungen wie zum Beispiel Magenschwüre verantwortlich sind (siehe zum Beispiel A. T. R. Axon, "Helicobacter pylori Infection", J. Antimicrob. Chemother. 32, Suppl. A, 61, 1993). Die Infektion des menschlichen Magens mit dem pathogenen gram-negativen Bakterium Helicobacter pylori ruft ein vorübergehendes dyspeptisches Beschwerdebild hervor, der Mikroorganismus hat aber eine hohe Persistenz. H. pylori ist zudem der zugrundeliegende Erreger bei der chronisch-aktiven Typ-b-Gastritis und ein signifikanter Risikofaktor für das Auftreten von Magenkrebs. Die pathophysiologischen Mechanismen, durch welche H. pylori Krankheiten des Magens hervorruft, sind noch relativ unklar. Es ist bekannt, daß der Mikroorganismus eine Reihe von potentiell toxischen Enzymen und Chemikalien produziert (Urease, Ammoniak, vakuolisierendes Cytotoxin). Die Persistenz des schädigenden Bakteriums und der dauernde antigene Stimulus sind wahrscheinlich ursächlich für die langfristig erfolgende Zerstörung der Magenschleimhaut.

Das therapeutische Ziel ist die vollständige Eradikation von H. pylori im Magen. Therapie der Wahl ist derzeit eine Dreifach-Kombination, die aus einem sogenannten Säurehemmer, zum Beispiel einem Protonenpumpenhemmer wie Omeprazol, und zwei Antibiotika, wie zum Beispiel Clarithromycin und Amoxicillin, besteht. Diese Tripeltherapie ist jedoch mit Nachteilen verbunden. Die drei unterschiedlichen Stoffe, die zusammenwirken sollen, erreichen aufgrund verschiedener Diffusionseigenschaften nicht gleichmäßig die von H. pylori verursachten Entzündungsherde. So sind, um gute Heilungserfolge zu erzielen, sehr hohe Dosierungen erforderlich, die mit ernsten Nebenwirkungen einhergehen. Es liegt auf der Hand, daß eine Tripeltherapie auch sonst generell große Nachteile hat im Vergleich zum Verabreichen von zwei Medikamenten oder gar nur eines Medikaments.

In der EP-A-655 249 wurde bereits beschrieben, daß Moenomycin-Antibiotika ebenfalls als wirksame Antibiotika zur Bekämpfung von H. pylori-Infektionen und somit zur Behandlung von Magenerkrankungen wie zum Beispiel Magengeschwüren geeignet sind. Auch die Moenomycin-Antibiotika entfalten ihre Wirkung gegen H. pylori besonders vorteilhaft bei Verabreichung in Kombination mit anderen Antibiotika oder anderen üblichen Ulcustherapeutika oder Gastritistherapeutika, zum Beispiel mit Antacida, H₂-Rezeptorblockern, Protonenpumpenhemmern, Muscarinrezeptorblockern oder zum Beispiel auch mit Wismutsalzen, wie zum Beispiel Wismutnitrat, Wismutcarbonat, Wismutsalicylat oder Wismutcitrat.

Über die Anwendung von Wismutsalzen zur Behandlung von Magen-Ulcera ist in der Literatur vielfach berichtet worden (siehe zum Beispiel J. H. Walsh und W. L. Peterson, N. Eng. J. Med. 333 (Nr. 15), 984-991, 1995). Als Wismutverbindungen dazu werden insbesondere basische Wismutverbindungen eingesetzt, unter anderem zum Beispiel das Wismutsubsalicylat, oder auch das Trikaliumsalz des Dicitratobismutats. Die Wirksamkeit des Wismuts wird auf der einen Seite auf dessen adstringierende Eigenschaft zurückgeführt, auf der anderen Seite wurden aber auch direkte Effekte des Wismuts auf Helicobacter pylori beschrieben, wie zum Beispiel die Hemmung der F1-ATPase aus H. pylori (W. Beil und C. Birkholz, Arch. Pharmacol. 350, Suppl. R 1, 1994). Der Einsatz von Wismutsalzen insbesondere bei der Behandlung von Helicobacter pylori-induzierten Magenerkrankungen wird deshalb angestrebt.

Leider stehen der Wismutanwendung in der bisherigen Form einige Nachteile entgegen, die im chemischen Verhalten der Wismutsalze begründet sind. Wismutsalze sind sehr häufig im wäßrigen Milieu wenig löslich. Aus dem Bi³⁺-Ion entstehen im wäßrigen Milieu sogenannte basische Salze, die das BiO¹⁺-Ion enthalten und die auch als Wismutsubsalze bezeichnet werden. Solche basischen Wismutsalze fallen aus wäßriger Lösung aus, d. h. sie bilden schwerlösliche Niederschläge, und stehen somit für eine biologische Wirkung nicht oder nur eingeschränkt zur Verfügung. Die gelösten Anteile bilden Kolloide. Aufgrund dieser physikochemischen Eigenschaften haben Wismutsalze oft nur angenähert angebbare Zusammensetzungen (siehe Römpps Chemie Lexikon, 9. Auflage, Georg Thieme Verlag, Stuttgart, New York, 1989, S. 439; DAB (Deutsches Arzneibuch) 8, 526-530) und haben eine schlechte Dosierbarkeit, die die Abschätzung ihrer Wirkung zusätzlich erschwert.

Die angestrebte Verabreichung von Wismutsalzen in Kombination mit zum Beispiel Säurehemmern und Antibiotika für die Behandlung von Magenerkrankungen wie Ulcera und von H. pylori-Infektionen erweist sich somit wegen der von den Säurehemmern und Antibiotika abweichenden Löslichkeit und Pharmakodynamik der Wismutsalze als sehr problematisch. Es hat deshalb nicht an Versuchen gefehlt, entsprechende Tripeltherapien zu Dualtherapien oder Monotherapien zu vereinfachen. Eine große Anzahl von Kombinationen aus Protonenpumpenhemmern, Antibiotika und Wismutsalzen wurde untersucht, ohne letztlich jedoch die Tripeltherapie übertreffen zu können. Es besteht somit weiterhin Bedarf an wirksamen, einfach anwendbaren und gut verträglichen Medikamenten für die Behandlung von Magenerkrankungen wie Magengeschwüren oder für die Prophylaxe von Magenkrebs.

Überraschend wurde nun gefunden, daß aus den Antibiotika der Moenomycin-Gruppe, die an ihren sauren Gruppen wie den Phosphorsäuregruppierungen (bzw. Phosphorsäureestergruppierungen) und/oder den Carbonsäuregruppierungen zur Salzbildung befähigt sind, stabile wohldefinierte Wismutsalze erhältlich sind, die die in Form von Einzelverbindungen oder von Gemischen vorliegende Antibiotikakomponente und das Wismut in stöchiometrischen Verhältnissen enthalten. Die Wirkung dieser Salze bei der Bekämpfung von Helicobacter pylori bzw. bei der Behandlung und Prophylaxe von Magenerkrankungen ist der Wirkung der reinen Antibiotika aus der Moenomycin-Gruppe deutlich überlegen ist und sie können in Form einer Dualtherapie oder sogar einer Monotherapie eingesetzt werden. Das Auffinden derartiger wohldefinierter Wismutsalze der Antibiotika der Moenomycin-Gruppe ist umso überraschender, als die herkömmlichen Verfahren zur Gewinnung solcher Salzen, wie das Fällen aus wäßriger Lösung, Dialysen oder lonenaustauscher-Anwendungen, an der bereits erörterten geringen Löslichkeit der Ausgangswismutsalze ebenso scheitern wie das Neutralisieren der freien Säuren der Antibiotika der Moenomycin-Gruppe mit basischem Wismut, zum Beispiel Wismuthydroxid.

Gegenstand der vorliegenden Erfindung sind somit Wismutsalze von einzeln oder im Gemisch vorliegenden Antibiotika der Moenomycin-Gruppe , und ihre physiologisch verträglichen Salze. Die erfindungsgemäßen Wismutsalze sind stöchiometrische Verbindungen, also definierte chemische Verbindungen von salzartigem Charakter, die das oder die sauren, in den erfindungsgemäßen Salzen in anionischer Form vorliegenden Antibiotika und das Wismut in bestimmten stöchiometrischen Verhältnissen enthalten. Sie sind Wismut(III)-Salze, sie sind jedoch keine basischen Wismutsalze und weisen nicht deren oben erläuterte Nachteile auf.

Das stöchiometrische Verhältnis, in dem das Wismut und das oder die Antibiotika in den erfindungsgemäßen Salzen vorliegen, hängt zum Beispiel von der Zahl der sauren Gruppen in dem oder in den Antibiotika- Molekülen ab und kann durch die angewandten Herstellungsbedingungen, zum Beispiel durch das bei der Herstellung eingesetzte Molverhältnis der Ausgangsverbindungen, eingestellt werden. Eine charakteristische Struktureinheit in Antibiotika der Moenomycin-Gruppe ist die Phosphoglycerinsäuregruppierung oder, als Teil davon, die zweifach veresterte Phosphorsäuregruppierung, an deren freier Säurefunktion eine Salzbildung stattfinden kann und die dann eine Bindungsstelle des Wismuts darstellt. Die Angabe des stöchiometrischen Verhältnisses in den erfindungsgemäßen Salzen kann zum Beispiel erfolgen durch die Angabe der Molzahl oder Atomzahl des Wismuts, die pro Mol des oder der Antibiotika vorhanden ist, oder sie kann in einfacher Weise zum Beispiel auch erfolgen durch die Angabe des leicht zu bestimmenden Phosphor-Wismut-Molverhältnisses bzw. -Atomverhältnisses. Dieses Verhältnis kann beispielsweise 1 : 1 betragen oder 1 : 2 (im letzten Fall heißt das, daß auf zwei Phosphoratome oder Phosphorsäuregruppierungen in einem erfindungsgemäßen Salz ein Wismutatom vorhanden ist). Das Verhältnis kann aber auch andere Werte annehmen, auch nicht ganzzahlige Werte, zum Beispiel wenn sich das erfindungsgemäße Salz von einem Gemisch von zwei oder mehr unterschiedlichen Antibiotika ableitet. Wird ein solches Verhältnis, zum Beispiel das Verhältnis 1 : 1, zur Charakterisierung eines erfindungsgemäßen Salzes angegeben, so kann natürlich in makroskopischen Proben, wie sie zum Beispiel bei der technischen Produktion der erfindungsgemäßen Salze anfallen, der experimentell bestimmte Wert zum Beispiel aufgrund von schwankenden Antibiotikaverhältnissen oder von enthaltenen Nebenkomponenten geringfügig vom angestrebten Idealwert (zum Beispiel 1 : 1) abweichen und streuen, die Angabe eines solchen stöchiometrischen Verhältnisses für eine erfindungsgemäße Substanz umfaßt also natürlich auch unwesentlich davon abweichende Verhältnisse.

Zahlreiche Antibiotika aus der Moenomycin-Gruppe enthalten neben der genannten Phosphorsäuregruppierung eine oder gegebenenfalls auch mehrere Carbonsäuregruppierungen, an denen ebenfalls eine Salzbildung mit dem Wismut erfolgen kann. Enthalten die Antibiotika zum Beispiel insgesamt zwei Säurefunktionen im Molekül, wie zum Beispiel die häufig vorkommenden Vertreter mit einer sauren HO-Gruppe in der Phosphorsäureeinheit und mit einer COOH-Gruppe, so können sie zwei Äquivalente Wismut binden bzw. zwei (Salz-)Bindungen mit dem Wismut eingehen. Die dritte Valenz des ja dreiwertigen Wismuts kann dann zum Beispiel durch eine (Salz-) Bindung zu zusätzlichen Anionen (bzw. Anion-Äquivalenten) abgesättigt werden, welche dann neben den Antibiotika und dem Wismut in den erfindungsgemäßen Salzen enthalten sind und welche zum Beispiel aus dem bei der Herstellung der erfindungsgemäßen Salze verwendeten Ausgangswismutsalz stammen können. Die dritte Valenz des Wismuts kann aber auch durch eine (Salz-)Bindung zu einer Phosphorsäuregruppierung oder Carbonsäuregruppierung in einem zweiten Molekül des Antibiotikums abgesättigt werden, wobei letztlich ein erfindungsgemäßes Salz resultiert, das zwei Wismutatome auf drei Moleküle der bezeichneten Antibiotika oder auf drei Phosphoratome enthält. Entsprechend können auch bei anderen Antibiotika und ganz allgemein bei den erfindungsgemäßen Verbindungen Valenzen des Wismuts durch zusätzliche Anionen abgesättigt sein. Als solche zusätzlichen Anionen kommen insbesondere physiologisch verträgliche Anionen in Betracht, zum Beispiel Chlorid, Bromid, Nitrat, Sulfat, Phosphat, und andere in Arzneimitteln einsetzbare anorganische und organische Anionen wie Acetat, Benzoat, Citrat, Tartrat, Methansulfonat, etc. In den erfindungsgemäßen Salzen können auch mehrere solcher zusätzlichen Anionen im Gemisch vorliegen.

Bevorzugt sind erfindungsgemäße Wismutsalze, die aufgrund ihres stöchiometrischen Verhältnisses Wismut: Antibiotikum zur Absättigung des Wismuts ein zusätzliches physiologisch verträgliches Anion enthalten, insbesondere eines der vorstehend genannten Anionen. Diese Salze können angesehen werden als kationischer Komplex aus dem Wismut und dem oder den Antibiotika, zu dem das physiologisch verträgliche Anion das negative Gegenion darstellt. Besonders bevorzugt sind erfindungsgemäße Wismutsalze, in denen das Wismut und das einzeln oder im Gemisch vorliegende Antibiotikum (bzw. das Wismut und der Phosphor in den teilveresterten Phosphorsäuregruppierungen) in einem Molverhältnis bzw. Atomverhältnis von 1 : 1 (oder ungefähr 1 : 1) vorliegen und die ein zusätzliches physiologisch verträgliches Anion enthalten, wobei dieses Anion ein einfach geladenes Anion sein kann oder ein Äquivalent eines mehrfach geladenen Anions sein kann. Diese besonders bevorzugten Salze können durch die Formel [A Bi]⁺ X⁻ dargestellt werden, in der A für ein einzeln oder im Gemisch vorliegendes Antibiotikum der Moenomycin-Gruppe steht, das zwei saure, in anionischer Form vorliegende Gruppen enthält, und X⁻ für ein physiologisch verträgliches einfach geladenes Anion oder ein Äquivalent eines physiologisch verträglichen mehrfach geladenen Anions steht.

Die Antibiotika der Moenomycin-Gruppe sind Phosphoglycolipid-Antibiotika. An Stelle des hier gebrauchten Begriffs Antibiotika der Moenomycin-Gruppe wird für diese Verbindungen teilweise auch der Begriff Phosphoglycolipid-Anitibiotika gebraucht. Die vorliegende Erfindung umfaßt die Wismutsalze aller Antibiotika dieser Gruppe. Insbesondere sind unter Antibiotika der Moenomycin-Gruppe zu verstehen die eigentlichen Moenomycine, d. h. das Moenomycin selbst, und zum Beispiel Prasinomycin, Diumycin (Macarbomycin), 11837 R.P., 8036 R.P. (Quebemycin), 19402 R.P., Ensachomycin, Prenomycin, Teichomycin, Pholipomycin und andere, die alle verwandte phosphorhaltige saure Glycolipide sind. Bevorzugt sind die Wismutsalze des Moenomycins selbst. Die einzelnen Antibiotika der Moenomycin-Gruppe sind vielfach wiederum Komplexe von mehreren, sich strukturell unterscheidenden Einzelkomponenten. Als Einzelkomponenten des Moenomycins selbst seien zum Beispiel genannt die Moenomycine A, A_{1.1}, A_{1.2}, B₁, B₂, C₁, C₂, C₃, C₄ und andere, wobei zum Teil auch abweichende Bezeichnungsweisen gebräuchlich sind, beispielsweise A_{1/2}. Bevorzugte Einzelkomponenten des Moenomycins selbst bezüglich der vorliegenden Erfindung sind das Moenomycin A (Formel I) und das Moenomycin C₃ (Formel II) (in den Formeln steht Ac für Acetyl). Eine besonders bevorzugte Einzelkomponente ist das Moenomycin A. Hinsichtlich weiterer Angaben zu den Antibiotika der Moenomycin-Gruppe und speziell dem Moenomycin selbst und seinen Einzelkomponenten sowie weiteren Strukturformeln wird auf die Literatur verwiesen, zum Beispiel auf den Artikel von G. Huber in "Antibiotics", ed. F. Hahn, Springer-Verlag, Berlin 1979, Vol. V/1, Seite 135 ff., oder auf die EP-A-655 249 und die dazu korrespondierenden Patentanmeldungen, die insofern vollinhaltlich Bestandteil der vorliegenden Offenbarung sind.

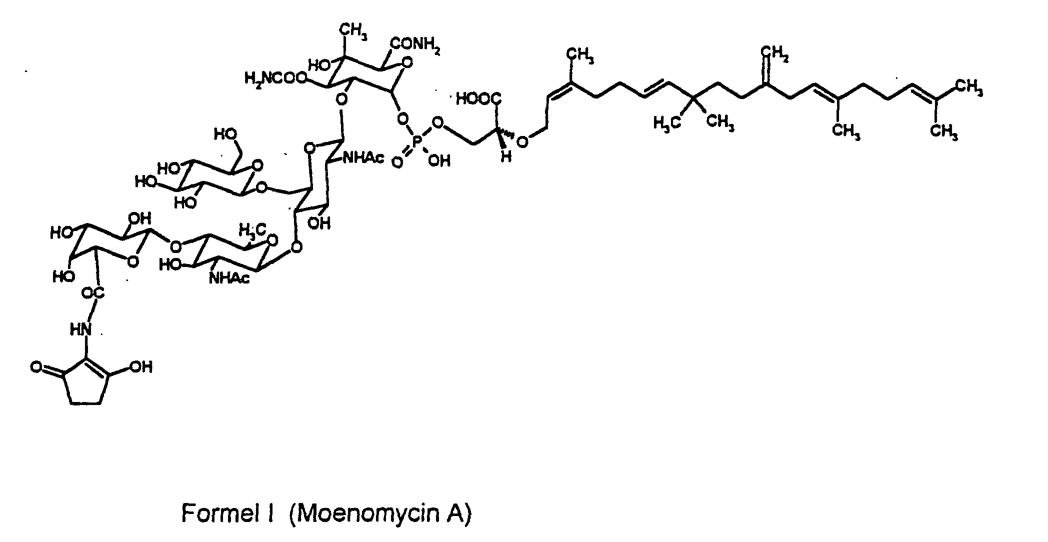

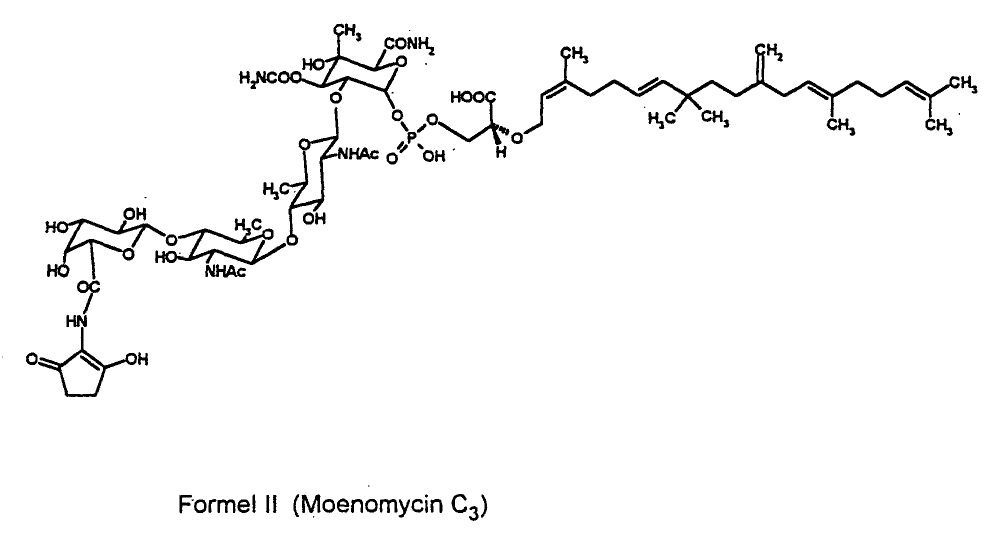

Die Antibiotika der Moenomycin-Gruppe und ebenso die eigentlichen Moenomycine werden im allgemeinen durch Fermentation von Mikroorganismen und nachfolgende Aufreinigung gewonnen. Eingesetzte Mikroorganismen sind zum Beispiel Streptomyces bambergensis, S. ghanaensis, S. ederensis, S. geysirensis, S. prasinus, S. lividoclavatus, und andere (siehe oben angegebene Literatur). Dabei fallen die Antibiotika vielfach als Gemische oder als Komplexe von Einzelkomponenten mit gegebenenfalls variierender Zusammensetzung an und werden vielfach auch in Form von solchen Gemischen verwendet. Gewünschtenfalls können die Gemische nach üblichen Methoden in die reinen oder weitgehend reinen Einzelantibiotika bzw. Einzelkomponenten aufgetrennt werden, die definierte Wirksamkeiten aufweisen und bevorzugt verabreicht werden. Entsprechend können sich auch die erfindungsgemäßen Wismutsalze von Gemischen von Antibiotika der Moenomycin-Gruppe ableiten oder von einzelnen Antibiotika oder von Einzelkomponenten der Komplexe. Die Wismutsalze aller Einzelantibiotika aus der Moenomycin-Gruppe und alle möglichen Zusammenstellungen mehrerer Antibiotika der Moenomycin-Gruppe werden von der vorliegenden Erfindung umfaßt. Die Gemische können sich von zwei oder mehr einzelnen Antibiotika ableiten, wobei es sich bei diesen um Einzelkomponenten eines bestimmten Antibiotikums handeln kann, zum Beispiel des Moenomycins selbst, und/oder diese zu unterschiedlichen Antibiotika aus der Moenomycin-Gruppe gehören können. In den erfindungsgemäßen Salzen enthaltene Gemische von Antibiotika können die Zusammensetzung aufweisen, in der sie bei ihrer Synthese oder Aufreinigung angefallen sind, oder auch zum Beispiel zur Erzielung eines bestimmten Wirkprofils durch gezieltes Vermischen von zwei oder mehr Einzelantibiotika oder Ausgangsgemischen hergestellt werden.

Gegenstand der vorliegenden Erfindung sind auch die physiologisch verträglichen Salze der Wismutsalze der Antibiotika der Moenomycin-Gruppe. Als Salze können bereits die oben erläuterten erfindungsgemäßen Verbindungen angesehen werden, die zusätzliche Anionen wie Chlorid, Bromid, Nitrat etc. enthalten. Enthält ein erfindungsgemäßes Wismutsalz als zusätzliches Anion ein Anion einer mehrbasigen Säure, zum Beispiel Schwefelsäure oder Citronensäure, so kann auch nur eine dieser Säurefunktionen durch Wismut abgesättigt sein und die zweite oder weitere können ganz oder teilweise zum Beispiel als Metallsalze oder Ammoniumsalze vorliegen. Auch können im Molekül des Antibiotikums vorhandene oder durch Derivatisierung erzeugte saure Gruppen, die nicht durch Salzbildung mit dem Wismut abgesättigt sind, als Metallsalze oder Ammoniumsalze vorliegen, oder es können in den Antibiotika vorhandene oder durch Derivatisierung, zum Beispiel durch Hydrolyse von Amidgruppen zu Aminogruppen, erzeugte basische Gruppen als Säureadditionssalze vorliegen. Als Metallsalze kommen insbesondere Alkalisalze und Erdalkalimetallsalze in Betracht, zum Beispiel Natrium-, Kalium-, Calcium- oder Magnesiumsalze. Ammoniumsalze können sich vom Ammoniak und von organischen Aminen ableiten. Säureadditionssalze können sich zum Beispiel von Chlorwasserstoffsäure, Bromwasserstoffsäure, Salpetersäure, Schwefelsäure, Phosphorsäure oder anderen in Arzneimitteln einsetzbaren anorganischen und organischen Säuren, wie Essigsäure, Benzoesäure, Citronensäure, Weinsäure, Methansulfonsäure und anderen, ableiten. Die Herstellung der Salze kann nach den üblichen, dem Fachmann bekannten Verfahren erfolgen. Auch innere Salze (Betaine) werden von der Erfindung umfaßt. Gegenstand der vorliegenden Erfindung sind weiterhin Solvate der Wismutsalze von Antibiotika der Moenomycin-Gruppe, zum Beispiel mit Wasser oder Alkoholen, und andere Derivate, zum Beispiel Ester, und aktive Metabolite der erfindungsgemäßen Verbindungen.

Die erfindungsgemäßen Wismutsalze der Antibiotika der Moenomycin-Gruppe sind zum Beispiel erhältlich, indem man das oder die Antibiotika der Moenomycin-Gruppe oder insbesondere deren üblicherweise verwendete Salze in einem Lösungsmittel oder Dispergiermittel mit einem Wismutsalz umsetzt. Dieses Herstellverfahren ist ebenfalls Gegenstand der vorliegenden Erfindung. Die Antibiotika werden dabei üblicherweise in Form von Alkalimetallsalzen oder Ammoniumsalzen, bevorzugt in Form der Natrium-, Kalium- oder Ammoniumsalze, eingesetzt, die in organischen Lösungsmitteln ausreichend löslich sind. Als Lösungsmittel für die Umsetzung werden bevorzugt organische Lösungsmittel verwendet, es können aber auch organische Lösungsmittel mit einem Wasseranteil verwendet werden. Bevorzugt werden die Antibiotika (bzw. ihre Ausgangssalze) und das Ausgangswismutsalz in Form von Lösungen eingesetzt, besonders bevorzugt wird insbesondere das Ausgangswismutsalz in Form einer Lösung in einem organischen Lösungsmittel eingesetzt. Bei der Umsetzung bilden sich definierte, stöchiometrisch zusammengesetzte Wismutsalze der Antibiotika der Moenomycin-Gruppe, die vielfach in organischen Lösungsmitteln schwerlöslich sind und bereits in weitgehend reiner Form aus dem Reaktionsgemisch ausfallen und in einfacher Weise abgetrennt werden können, oder die sonst nach üblichen Methoden isoliert und gewünschtenfalls weiter gereinigt werden können.

Die Bestimmung der Zusammensetzung der so erhältlichen erfindungsgemäßen Produkte und der Nachweis, daß es sich nicht um physikalische Mischungen von Wismutverbindungen mit den Antibiotika handelt, sondern um homogene, stöchiometrisch zusammengesetzte chemische Verbindungen, die das Wismut, die Antibiotika und gegebenenfalls zum Beispiel ein zusätzliches Anion enthalten, kann nach den üblichen, dem Fachmann bekannten Analysenverfahren erfolgen. Der Gehalt an Wismut und anderen Elementen kann zum Beispiel durch Elementaranalyse in bekannter Weise bestimmt werden. Den Gehalt eines erfindungsgemäßen Salzes an Antibiotika kann man zum Beispiel durch Aufnahme von NMR-Spektren nachweisen und identifizieren.

Die NMR-Spektroskopie erlaubt auch eine eindeutige Aussage, daß eine definierte Wismutsalzbildung an bestimmten sauren Gruppen des Antibiotikums erfolgt. Zum Beispiel zeigt ein Vergleich der ¹H- und ¹³C-NMR-Signale eines erfindungsgemäßen Wismutsalzes mit denen des entsprechenden, als Ausgangsmaterial für seine Synthese verwendeten Natriumsalzes des Antibiotikums, daß in charakteristischer Weise bestimmte Signale verschoben sind, insbesondere solche von Atomen, die den sauren Zentren benachbart sind, an denen die Wismutsalzbildung erfolgt (vgl. NMR-Daten im Beispiel 1). Dies belegt, daß die erfindungsgemäßen Substanzen neue, wohldefinierte Verbindungen darstellen und bestätigt ihre Struktur.

Gut geeignet sind auch röntgenspektrometrische Verfahren wie die Röntgenfluoreszenzanalyse und darüber hinaus die Rastertransmissions-Elektronenmikroskopie, die mit energiedispersiven Röntgenmikroanalysen gekoppelt werden kann. Diese Analysenmethoden erlauben neben dem Nachweis der Elemente, zum Beispiel des Wismuts oder des Phosphors, die Bestimmung des Verhältnisses der vorliegenden Elemente an unterschiedlichen, extrem kleinen Probenstellen, wie zum Beispiel das Verhältnis Wismut zu Phosphor, oder zum Beispiel zu Chlor, Natrium und anderen Elementen. Die erfindungsgemäßen Produkte sind auch im Sinne der energiedispersiven Röntgenmikroanalyse homogen, das Konzentrationsverhältnis von zum Beispiel Wismut zu Phosphor ist stets konstant, es liegt also eine stöchiometrisch zusammengesetzte Verbindung vor.

Bevorzugte Lösungsmittel bei der Herstellung der erfindungsgemäßen Wismutsalze sind, wie bereits gesagt, organische Lösungsmittel. Es können aber auch wäßrigorganische Lösungsmittel eingesetzt werden, wobei der Wassergehalt so gering sein sollte, daß unter den Reaktionsbedingungen keine nennenswerte Hydrolyse des als Ausgangsmaterials eingesetzten Wismutsalzes eintritt. Welcher Wassergehalt zulässig ist, hängt vom Einzelfall ab. Geeignete organische Lösungsmittel sind zum Beispiel niedere Alkohole, insbesondere Methanol und Ethanol, Ethylenglykolmonomethylether, Glykole, wie zum Beispiel Ethylenglykol oder 1,2-Propylenglykol, Dimethylsulfoxid (DMSO), Dimethylformamid, Ether wie Dioxan, Tetrahydrofuran, Ethylenglykolether, sowie Mischungen dieser Lösungsmittel und andere Lösungsmittel. Bevorzugt sind insbesondere niedere Alkohole, in denen sich die Antibiotika der Moenomycin-Gruppe bzw. ihre Salze gut lösen, wie Methanol oder Ethanol.

Bevorzugt werden zur Herstellung der erfindungsgemäßen Verbindungen die Antibiotika der Moenomycin-Gruppe als Lösung, zum Beispiel in Methanol, mit einer Konzentration von üblicherweise 0.1 bis 10 Gew.-%, vorzugsweise von 1 bis 5 Gew.-%, vorgelegt. Wie bereits gesagt, können die Antibiotika in Form von Gemischen mehrerer Antibiotika oder in in Form von Komplexen eines Antibiotikums, zum Beispiel des Moenomycins, oder als Einzelkomponenten, zum Beispiel als Moenomycin A oder C₃, in die Wismutsalzbildung eingesetzt werden, wobei, wie ebenfalls bereits gesagt, üblicherweise Salze wie zum Beispiel die Natriumsalze oder Ammoniumsalze eingesetzt werden. Geeignete Wismut(III)salze sind insbesondere Salze des Bi³⁺-Ions, die sich gut in organischen Lösungsmitteln lösen, wie zum Beispiel Wismutchlorid (BiCl₃), Wismutnitrat (Bi(NO₃)₃), Wismutbromid (BiBr₃) und andere. Die eingesetzte Wismut(III)-Salzlösung in einem organischen Lösungsmittel hat üblicherweise eine Konzentration von 0.01 bis 1 Mol pro Liter, vorzugsweise von 0.1 bis 0.5 Mol pro Liter. Die Wismutlösung wird bevorzugt zur Lösung des Antibiotikums zudosiert, wobei vielfach, aber natürlich abhängig vom Einzelfall, bereits das erfindungsgemäße Salz ausfällt. Das molare Verhältnis der Reaktanten kann in weitem Bereich variiert werden. Bevorzugt verwendet man äquimolare Verhältnisse, insbesondere, wenn die bevorzugten Wismutsalze hergestellt werden sollen, die das Antibiotikum (bzw. den Phosphor) und das Wismut im Molverhältnis bzw. Atomverhältnis 1 : 1 enthalten. Wie bereits gesagt, können je nach den angewandten Herstellungsbedingungen verschiedene Wismutsalze erhalten werden.

Im allgemeinen erfolgt die Umsetzung der Wismutsalze mit den Antibiotika im Temperaturbereich von -20 °C bis 80 °C, vorzugsweise im Bereich von 10 °C bis 30 °C. Die Umsetzung wird vorteilhafterweise durch langsames Zudosieren im Verlauf von im allgemeinen 20 Minuten bis 2 Stunden durchgeführt. Bei rascherer Arbeitsweise können, wenn das Produkt ausfällt, sogenannte Einschlüsse die Reinheit des Produktes beeinträchtigen. Ist das Produkt in dem eingesetzten Lösungsmittel schwer löslich, so kann zur Isolierung des Produkts der bei der Ausfällung entstandene Niederschlag durch Zentrifugieren oder Filtrieren abgetrennt und, wenn gewünscht, zum Beispiel durch Aufschlämmen in einem geeigneten organischen Lösungsmittel und erneutes Zentrifugieren oder Filtrieren gereinigt werden. Ist das Produkt leicht löslich, so daß es vollständig oder zu einem größeren Teil in Lösung verbleibt, so kann nach den üblichen Vorgehensweisen das Lösungsmittel zunächst teilweise oder ganz entfernt werden, zum Beispiel durch Vakuumdestillation und/oder Gefriertrocknung, und/oder ein Lösungsmittel zugesetzt werden, in dem das Produkt schwer löslich ist, und dann die Abtrennung des ausgefällten Produkts erfolgen. Zur Isolierung können aber auch chromatographische Verfahren eingesetzt werden. Nach Trocknung erhält man das Produkt als ein weißes oder helles Pulver, welches im allgemeinen gut löslich in Wasser und löslich in DMSO, aber schwer löslich in vielen anderen organischen Lösungsmitteln ist. Gewünschtenfalls kann das Produkt noch nach üblichen Verfahren, zum Beispiel durch Umfällen oder Chromatographie, gereinigt werden.

Weiterhin können insbesondere erfindungsgemäße Wismutsalze, die ein zusätzliches Anion enthalten, zum Beispiel durch lonenaustauschverfahren erhalten werden. So kann zum Beispiel ein erfindungsgemäßes Wismutsalz, das ein bestimmtes Anion enthält, durch Anionenaustausch nach den üblichen Vorgehensweisen, zum Beispiel durch Umsetzung mit einem Salz oder einer Säure in einem Lösungsmittel oder durch Chromatographie, in ein anderes erfindungsgemäßes Wismutsalz überführt werden, das ein anderes physiologisch verträgliches Anion als zusätzliches Anion enthält.

Die biologische und therapeutische Wirkung der Antibiotika der Moenomycin-Gruppe, insbesondere der Moenomycine selbst, sowie die Vorteile des Einsatzes dieser Antibiotika in der Therapie und Prophylaxe von Magenerkrankungen sind in der EP-A-655 249 ausführlich beschrieben. Die erfindungsgemäßen Wismutsalze übertreffen die vorteilhaften antibakteriellen und heilungsfördernden Eigenschaften der Moenomycine sogar noch in erheblichem Maß. Das zeigt sich sowohl in in vitro-Versuchen als auch in vivo. Wie weiter unten ausgeführt, ist beispielsweise die Wirkung des Wismutsalzes des Moenomycins A der Wirkung des Moenomycins A (als Natriumsalz) bereits in in vitro-Experimenten deutlich überlegen. Das Moenomycin und das Wismut wirken synergistisch. In Form der erfindungsgemäßen Salze können beide Komponenten zusammen durch Diffusion an den Entzündungsherd gelangen und dort ihre Wirkungen entfalten, was bei der üblichen Verabreichung eines Antibiotikums und eines separaten Wismutsalzes nicht oder nur in sehr viel geringerem Maße möglich ist.

Ein entscheidender Vorteil der erfindungsgemäßen Verbindungen ist, daß wegen der höheren Wirksamkeit der erfindungsgemäßen Verbindungen kleinere Dosen verabreicht werden können, um das therapeutische Ziel, zum Beispiel die H. pylori-Eradikation, zu erreichen, und daß damit weniger Nebenwirkungen verbunden sind. Die breite und hohe Wirksamkeit der erfindungsgemäßen Wismutsalze erlaubt ihren alleinigen Einsatz als antimikrobielles Agens an Stelle von zwei Antibiotika in der herkömmlichen Therapie. Damit kann die Tripeltherapie zur Dualtherapie vereinfacht werden, in der neben dem erfindungsgemäßen Wismutsalz zum Beispiel nur noch ein Säurehemmer (zum Beispiel Omeprazol, Lansoprazol, Pantoprazol oder andere) verabreicht wird. Der Patient wird dadurch geschont und die Kosten werden gesenkt. Es gelingt sogar die Eradikation von H. pylori mit den erfindungsgemäßen Wismutsalzen alleine ohne jede zusätzliche Medikation. Diese Monotherapie ist bezüglich Einfachheit, Verträglichkeit und Kostengünstigkeit anderen Ulcustherapien weit überlegen.

Die erfindungsgemäßen Wismutsalze von Antibiotika der Moenomycin-Gruppe und ihre physiologisch verträglichen Salze können somit am Tier, bevorzugt am Säugetier, und insbesondere am Menschen als Arzneimittel für sich allein, in Mischungen untereinander oder in Form von pharmazeutischen Präparaten verwendet werden. Gegenstand der vorliegenden Erfindung sind auch die erfindungsgemäßen Wismutsalze und ihre physiologisch verträglichen Salze zur Anwendung als Arzneimittel, ihre Verwendung in der Therapie und Prophylaxe von Ulcera allgemein, wie zum Beispiel Ulcus duodeni oder Ulcus pepticum, von Magenerkrankungen, insbesondere Magengeschwüren oder Gastritis, in der Prophylaxe von Magenkrebs, sowie generell bei der Bekämpfung von Helicobacter pylori, und ihre Verwendung zur Herstellung von Medikamenten für die genannten Verwendungen. Weiterhin sind Gegenstand der vorliegenden Erfindung pharmazeutische Präparate, die als aktiven Bestandteil eine wirksame Dosis mindestens eines erfindungsgemäßen Wismutsalzes und/oder eines physiologisch verträglichen Salzes davon neben üblichen, pharmazeutisch einwandfreien Trägerstoffen und/oder Hilfsstoffen enthalten. Die pharmazeutischen Präparate enthalten normalerweise 0,5 bis 95 Gewichtsprozent der erfindungsgemäßen Wismutsalze und/oder ihrer physiologisch verträglichen Salze. Die Herstellung der pharmazeutischen Präparate kann in an sich bekannter Weise erfolgen. Dazu werden die erfindungsgemäßen Wismutsalze und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann. Diese Arzneimittel sind vorwiegend zur oralen Applikation bestimmt.

Die erfindungsgemäßen Wismutsalze und ihre physiologisch verträglichen Salze können zur Erzielung einer vorteilhaften therapeutischen Wirkung auch mit anderen Arzneiwirkstoffen kombiniert werden, insbesondere mit einem oder mehreren weiteren Arzneimittelwirkstoffen zur Behandlung von Magenerkrankungen oder Ulcera. Für die genannten therapeutischen und prophylaktischen Verwendungen geeignete zusätzliche Wirkstoffe stammen zum Beispiel aus der Gruppe der Antacida, wie zum Beispiel Natriumhydrogencarbonat, Aluminiumhydroxid, Magnesiumhydroxid, Magnesiumtrisilikat, Aluminium-magnesium-silikat-hydrat, Aluminium-natrium-carbonat-dihydroxid, Magnesiumcarbonat, Calciumcarbonat oder Hydrotalcit. Weitere geeignete zusätzliche Wirkstoffe stammen aus der Gruppe der H₂-Rezeptorblocker, wie zum Beispiel Famotidin, Nizatidin, Roxatidinacetat, Ranitidin oder Cimetidin. Andere geeignete zusätzliche Wirkstoffe sind Muscarinrezeptorblocker wie Propanthelinbromid, Pirenzipin, oder andere Ulcusmittel wie Omeprazol, Lansoprazol, Pantoprazol, Misoprostol, oder auch zusätzliche Wismutsalze wie Wismutnitrat, Wismutcarbonat, Wismutsalicylat oder Wismutcitrat. Weitere für die erfindungsgemäße Therapie geeignete zusätzliche Wirkstoffe gehören zur Gruppe der Antibiotika, wie zum Beispiel Tetracyclin, Metronidazol, Amoxycillin, Nisin, Clarithromycin, Imipenem oder Amikacin. Eine bevorzugte Kombination enthält die erfindungsgemäßen Wismutsalze zusammen mit einem Protonenpumpenhemmer wie zum Beispiel Omeprazol, Lansoprazol, Pantoprazol oder anderen. Es kann auch vorteilhaft sein, die erfindungsgemäßen Wismutsalze mit mehreren der obengenannten zusätzlichen Wirkstoffe oder mit weiteren Wirkstoffen für andere Indikationen zu kombinieren. Die Verabreichung der Komponenten der genannten Kombinationen kann zusammen oder in getrennter Form erfolgen, und sie kann in einer einzigen Darreichung erfolgen oder auch in zeitlicher Abfolge vorgenommen werden.

Als galenische Applikationsformen für die Verabreichung der erfindungsgemäßen Wismutsalze kommen zum Beispiel in Betracht Kapseln, zum Beispiel harte oder weiche Kapseln, Tabletten, Pastillen, Lutschbonbons, Rollkuren, dispergierbare Pulver und Granulate, Mikroperlen, Lösungen oder Suspensionen, insbesondere wäßrige Lösungen und Suspensionen, Emulsionen, Sirupe und Elixiere, und ähnliche im Stand der Technik bekannte Formen. Feste Verabreichungsformen sind bevorzugt, insbesondere solche, die das wirksame Prinzip im Magen freisetzen.

Die pharmazeutischen Präparate können nach den einschlägigen, dem Stand der Technik entsprechenden Methoden für die Herstellung pharmazeutischer Präparate unter Verwendung pharmazeutisch akzeptabler, nicht-toxischer Hilfsstoffe und Trägerstoffe hergestellt werden. Tabletten für die orale Verabreichung können als Trägerstoffe beispielsweise inerte Streckmittel (wie etwa Natriumchlorid, Lactose, Calciumphosphat oder Natriumphosphat), Granulationsmittel oder Aufschlußmittel (zum Beispiel Kartoffelstärke, Alginsäure), Bindemittel (wie etwa Stärke, Gelatine oder Gummi Arabicum) und Schmiermittel (wie etwa Magnesiumstearat, Stearinsäure oder Talkum) enthalten. Die Tabletten können unbeschichtet sein oder sie können mittels der bekannten Techniken beschichtet sein, um die Auflösung und Resorption im Magen zu verzögern und somit eine anhaltende Wirkung über einen längeren Zeitraum zu bieten. So kann beispielsweise ein zeitverzögernder Stoff wie etwa Glycerylmonostearat oder Glyceryldistearat eingesetzt werden. In harten Gelatinekapseln für die orale Verabreichung kann der Wirkstoff beispielsweise gemischt sein mit einem inerten festen Streckmittel, beispielsweise Calciumphosphat oder Kaolin, in weichen Gelatinekapseln kann der Wirkstoff beispielsweise gemischt sein mit einem wäßrigen Medium, zum Beispiel Wasser, oder einem öligen Medium, beispielsweise Erdnußöl, flüssigem Paraffin oder Olivenöl. Die für die gewünschte Arzneimittelformulierung in Betracht kommenden Trägerstoffe und/oder Hilfsstoffe sind dem Fachmann auf Grund seines Fachwissens geläufig. Als Hilfsstoffe genannt seinen beispielsweise Antioxidantien, Dispergiermittel, Emulgatoren, Lösungsvermittler, Stabilisatoren, Geschmacksstoffe, Süßstoffe, Farbstoffe, Konservierungsmittel, Mittel zur Erzielung eines Depoteffekts, Puffersubstanzen, etc.

Die zu verabreichende Dosierung der erfindungsgemäßen Wismutsalze bzw. ihrer physiologisch verträglichen Salze hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Schwere der zu behandelnden Krankheit sowie von Geschlecht, Alter, Gewicht, Gesundheitszustand, Ernährung, individueller Ansprechbarkeit des zu behandelnden Menschen oder Tieres, von Wechselwirkungen mit anderen Arzneimitteln und davon, ob akut oder prophylaktisch behandelt wird. Üblicherweise beträgt die tägliche Dosis bei oraler Verabreichung eines pharmazeutischen Präparats an einen ungefähr 75 kg schweren Menschen 5 mg bis 5 g pro Person und Tag, vorzugsweise 50 mg bis 2 g pro Person und Tag. Die Dosis kann in Form einer Einzeldosis verabreicht werden oder in mehrere, zum Beispiel zwei, drei oder vier, Einzeldosen aufgeteilt werden.

Außer als Arzneimittelwirkstoffe können die erfindungsgemäßen Wismutsalze auch, wie bereits oben erwähnt, als Zwischenprodukte zur Herstellung weiterer Arzneimittelwirkstoffe verwendet werden. Ferner können sie als Hilfsmittel in biochemischen oder mikrobiologischen Untersuchungen oder in Diagnoseverfahren, zum Beispiel in in vitro-Diagnosen, eingesetzt werden.

### Beispiele

### Beispiel 1

### Wismutsalz des Moenomycins A in der Chlorid-Form (Formel: [C₆₉H₁₀₆BiN₅O₃₄P]⁺, Gegenion: Cl⁻; MG 1825)

50 g Moenomycin A-Natrium-Salz wurden in 2 l Methanol gelöst und während 2 Stunden bei Raumtemperatur mit 9.6 g BiCl₃ in 100 ml Methanol unter Rühren versetzt. Nach weiteren 15 Minuten gelindem Rühren wurde der entstandene weiße Niederschlag mehrmals mit je 2 l Methanol gewaschen, indem man mit einem Glasstab das Präzipitat aufrührte und das ungelöste Wismutsalz des Moenomycins A durch Zentrifugieren sammelte. Nach Trocknen im Vakuum wurden 28 g Produkt erhalten, das nach Mörsern und Sieben für die biologischen Untersuchungen eingesetzt wurde. Das erhaltene Produkt wurde unter anderem durch die folgenden Analysen charakterisiert.

### a) ¹H- und ¹³C-NMR

Chemische Verschiebungen (in ppm) des gemäß Beispiel 1 erhaltenen Moenomycin A-Wismutsalzes in der Chlorid-Form und - als Vergleich - des eingesetzten Moenomycin A-Natriumsalzes (in D₆-DMSO)

| Molekülposition (siehe Formel la unten) | Bi-Salz, Chlorid-Form | Na-Salz | Bi-Salz, Chlorid-Form | Na-Salz |
|---|---|---|---|---|
| | ¹H | ¹H | ¹³C | ¹³C |
| 1 | 4.07/3.94 | 4.07/3.83 | 65.42 | 64.78 |
| 2 | 5.33 | 5.32 | 121.66 | 123.51 |
| 3 | - | - | 138.73 | 136.84 |
| 4 | 2.06 | 2.03 | 31.80 | 31.96 |
| 5 | 2.05 | 2.02 | 30.70 | 30.88 |
| 6 | 5.25 | 5.24 | 125.23 | 125.43 |
| 7 | 5.36 | 5.35 | 139.80 | 139.79 |
| 8 | - | - | 35.07 | 35.19 |
| 9 | 1.33 | 1.33 | 40.86 | 40.91 |
| 10 | 1.86 | 1.85 | 30.61 | 30.68 |
| 11 | - | - | 149.15 | 149.21 |
| 12 | 2.66 | 2.65 | 34.39 | 34.47 |
| 13 | 5.12 | 5.11 | 121.66 | 121.75 |
| 14 | - | - | 135.69 | 135.81 |
| 15 | 1.98 | 1.98 | 39.08 | 39.22 |
| 16 | 2.05 | 2.05 | 26.01 | 26.08 |
| 17 | 5.07 | 5.06 | 123.96 | 124.05 |
| 18 | - | - | 130.56 | 130.70 |
| 19 | 1.63 | 1.63 | 25.34 | 25.48 |
| 20 | 1.55 | 1.55 | 17.37 | 17.53 |
| 21 | 1.57 | 1.57 | 15.62 | 15.71 |
| 22 | 4.66 | 4.65 | 108.58 | 108.70 |
| 23 | 0.94 | 0.93 | 27.02 | 27.11 |
| 24 | 0.94 | 0.93 | 27.02 | 27.11 |
| 25 | 1.70 | 1.67 | 23.20 | 23.28 |
| 26 | - | - | *) | 173.68 |
| 27 | 4.03 | 3.64 | 77.69 | 80.89 |
| 28 | 3.85 | 3.99/3.75 | 65.23 | 67.25 |
| A-NH | 8.66 | 7.47 | - | - |
| A1 | - | - | breit | 193.37 |
| A2 | - | - | *) | 109.67 |
| A3 | - | - | breit | 193.37 |
| A4 | breit | 2.00 | *) | 30.88 |
| A5 | breit | 2.00 | *) | 30.88 |
| B1' | 4.41 | 4.33 | 102.87 | 103.24 |
| B2' | 3.41 | 3.38 | 69.79 | 70.24 |
| B3' | 3.41 | 3.37 | 72.48 | 72.98 |
| B4' | 3.93 | 3.92 | 69.08 | 69.46 |
| B5' | 4.26 | 3.94 | 74.31 | 75.36 |
| B5'-C' | - | - | 169.37 | 167.03 |
| C1' | 4.51 | 4.66 | 101.09 | 100.98 |
| C2' | 3.52 | 3.52 | 55.50 | 55.63 |
| C2'-NH | 7.81 | 8.08 | - | - |
| C2'-C' | - | - | 169.44 | 170.03 |
| C2'-Ac | 1.88 | 1.90 | 22.94 | 22.97 |
| C3' | 3.54 | 3.48 | 71.66 | 72.89 |
| C4' | 3.21 | 3.15 | 83.71 | 84.02 |
| C5' | 3.52 | 3.48 | 70.32 | 70.73 |
| C5'-Me | 1.33 | 1.31 | 17.37 | 17.53 |
| D1' | 4.35 | 4.37 | 102.64 | 102.88 |
| D2' | 2.97 | 2.96 | 73.41 | 73.49 |
| D3' | 3.25 | 3.26 | 76.67 | 76.69 |
| D4' | 3.04 | 3.01 | 70.33 | 70.24 |
| D5' | 3.21 | 3.21 | 76.67 | 77.21 |
| D6' | 3.70/3.44 | 3.71/3.44 | 61.24 | 61.52 |
| E1' | 4.51 | 4.46 | 101.09 | 101.53 |
| E2' | 3.37 | 3.55 | 55.02 | 55.01 |
| E2'-NH | 7.41 | 7.61 | - | - |
| E2'-C' | - | - | 169.03 | 169.81 |
| E2'-Ac | 1.82 | 1.82 | 23.10 | 23.03 |
| E3' | 3.59 | 3.53 | 71.83 | 72.79 |
| E4' | 3.35 | 3.35 | 80.27 | 79.67 |
| E5' | 3.41 | 3.38 | 73.83 | 72.03 |
| E6' | 3.97/3.54 | 3.9913.44 | 67.39 | 68.28 |
| F1' | 5.67 | 5.75 | 93.60 | 93.69 |
| F2' | 3.43 | 3.38 | 76.63 | 77.21 |
| F3' | 4.91 | 4.90 | 73.85 | 74.15 |
| F3'-NH₂ | 6.25 | 6.36 | - | - |
| F3'-C' | - | - | 156.15 | 156.53 |
| F4' | - | - | 72.60 | 72.53 |
| F4'-Me | 1.10 | 1.06 | 16.21 | 16.04 |
| F5' | 4.21 | 4.28 | 71.61 | 71.71 |
| F5'-NH₂ | 7.4217.02 | 7.4917.29 | - | - |
| F5'-C' | - | - | 171.79 | 171.80 |

| | | | | |
|---|---|---|---|---|
| *) Diese Signale können nicht eindeutig zugeordnet werden. | | | | |

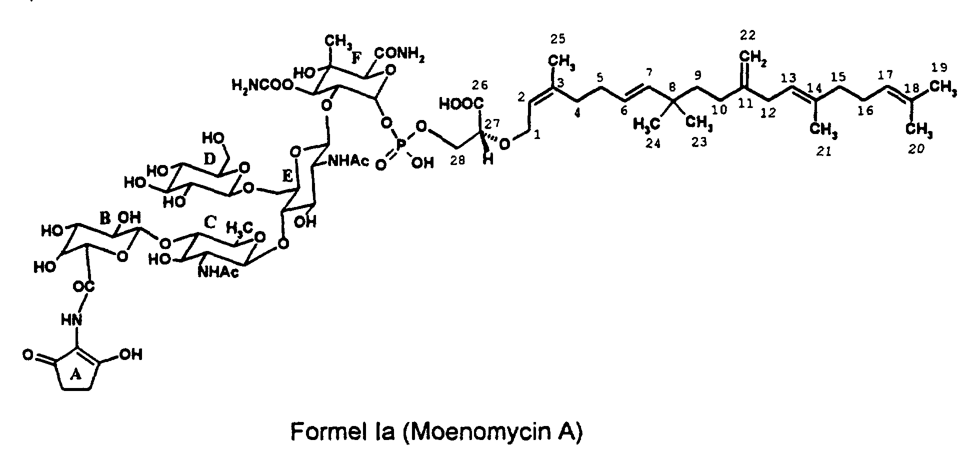

### b) Elektronendispersive Röntgenmikroanalyse (EDX)

Es wurden zehn Pulveragglomerate mittels EDX untersucht. Der Durchmesser der untersuchten Probenstellen betrug jeweils ca. 50 nm. An jeder Stelle wurde Wismut nachgewiesen. Das Atomverhältnis Wismut: Phosphor lag stets bei 1 : 1, d. h. das Wismut ist homogen und stöchiometrisch in den organischen Pulverteilchen eingebaut. Neben dem Wismut und Phosphor (sowie Chlor, Kohlenstoff und Sauerstoff) wurden in lokal unterschiedlichen Konzentrationen geringe Mengen Natrium nachgewiesen.

Der Überstand der Moenomycin A/Wismutchlorid-Fällung wurde mit 100 ml DMSO versetzt, im Vakuum auf 200 ml konzentriert und auf eine mit 20 l Fractogel TSK HW-40 gefüllte Säule aufgetragen. Es wurde mit DMSO/Methanol (1:1) fraktioniert eluiert. Die das Wismutsalz des Moenomycins A enthaltenden Eluat-Fraktionen wurden zusammengefaßt und durch Vakuumdestillation sowie durch Gefriertrocknung vom Lösungsmittel befreit. Sie ergaben weitere 21 g des Wismutsalzes des Moenomycins A in der Chlorid-Form, das mit dem zuerst erhaltenen Produkt identisch war.

### Beispiel 2

### Wismutsalz des Moenomycins A in der Nitrat-Form (Formel: [C₆₉H₁₀₆BiN₅O₃₄P]⁺, Gegenion: NO₃⁻; MG 1851.5)

163 mg Moenomycin A-Natriumsalz wurden in 4 ml Methanol gelöst und mit einer Lösung von 48.5 mg Wismut(III)nitrat-Pentahydrat in 200 µl DMSO unter Rühren versetzt. Es entstand ein Niederschlag, der durch dreimaliges Aufschlämmen in Methanol, Zentrifugieren und Abtrennen des Überstandes gereinigt wurde. Nach der Trocknung des Niederschlages im Vakuum wurden 112 mg Wismutsalz des Moenomycins A in der Nitratform erhalten. Die elektronendispersive Röntgenmikroanalyse wurde wie in Beispiel 1 durchgeführt und ergab entsprechende Ergebnisse, jedoch keine Chlor-Anteile.

Entsprechend den vorstehenden Beispielen können auch folgende Wismutsalze von Moenomycin-Antibiotika erhalten werden:
Moenomycin A_{1.2}-Wismutsalz in der Chlorid-Form
   (Formel: [C₆₈H₁₀₄BiN₅O₃₄P]⁺, Gegenion: Cl⁻; MG 1811)
Moenomycin C₁-Wismutsalz in der Chlorid-Form
   (Formel: [C₆₂H₉₄BiN₅O₂₈P]⁺, Gegenion: Cl⁻; MG 1632.8)
Moenomycin C₃-Wismutsalz in der Chlorid-Form
   (Formel: [C₆₃H₉₆BiN₅O₂₈P]⁺, Gegenion: Cl⁻; MG 1646.9)
Moenomycin C₄-Wismutsalz in der Chlorid-Form
   (Formel: [C₆₃H₉₆BiN₅O₂₉P]⁺, Gegenion: Cl⁻; MG 1662.9)

### Biologische Untersuchungen

### Antibakterielle Aktivität der Wismutsalze von Antibiotika der Moenomycin-Gruppe gegenüber Helicobacter pylori

Helicobacter pylori wurde auf Tryptic Soy-Agar (+5 % defibriniertes Schafblut, + Actidione 500 µg/ml) unter mikroaerophilen Bedingungen (Anaerocult, Merck) in CO₂-Atmosphäre (8 - 10 % CO₂) für 5 Tage bei 35 °C vorgezüchtet. Für den eigentlichen Versuch wurden die gewachsenen Kulturen mit einem Wattetupfer vollständig von der Vorzuchtplatte abgenommen, in 0.9 %iger NaCI-Lösung suspendiert und mit McFarland-Standard auf eine Keimdichte von 3 x 108 cfu/ml eingestellt. Die in vitro-Aktivität der Prüfsubstanzen wurde nach der Agardilutionsmethode mit Columbia-Agar (+5 % defibriniertes Schafblut, + Actidione, 500 µg/ml) als Testmedium bestimmt. Die Agarplatten, die verschiedene Konzentrationen an Prüfsubstanz enthielten (0.002 bis 128 µg/ml), wurden punktförmig (Multipoint-Inokulator, Denley) mit den eingestellten Keimsuspensionen beimpft. Die Inkubation erfolgte unter mikroaerophilen Bedingungen (s.o.). Nach 5 Tagen bei 35 °C wurde die geringste Substanzkonzentration ermittelt, bei der visuell keine Koloniebildung erkennbar war, und als die minimale Hemmkonzentration (MHK) definiert. Wie die Wismutsalze wurden zum Vergleich die entsprechenden Natriumsalze untersucht.

Ergebnisse: Minimale Hemmkonzentration (µg/ml)

| Keim | Moenomycin A-Natriumsalz (Vergleich) | Moenomycin A-Wismutsalz, Chlorid-Form (Beispiel 1) |
|---|---|---|
| H. pylori P 42 | 2 | 0.5 |

## Patentansprüche

1. Wismutsalze von einzeln oder im Gemisch vorliegenden Antibiotika der Moenomycin-Gruppe, und ihre physiologisch verträglichen Salze.

2. Wismutsalze gemäß Anspruch 1, **dadurch gekennzeichnet, daß** sie sich von einem oder mehreren der Antibiotika Moenomycin, Prasinomycin, Diumycin, 11837 R.P., 8036 R.P., 19402 R.P., Ensachomycin, Prenomycin, Teichomycin und Pholipomycin ableiten, und ihre physiologisch verträglichen Salze.

3. Wismutsalze gemäß Anspruche 1 und/oder 2, **dadurch gekennzeichnet, daß** sie sich von dem in Form einer Einzelkomponente oder in Form eines Gemisches von Einzelkomponenten vorliegenden Moenomycin selbst ableiten, und ihre physiologisch verträglichen Salze.

4. Wismutsalze gemäß einem oder mehreren der Ansprüche 1 bis 3, **dadurch gekennzeichnet, daß** sie sich vom Moenomycin A und/oder Moenomycin C₃ ableiten, und ihre physiologisch verträglichen Salze.

5. Wismutsalze gemäß einem oder mehreren der Ansprüche 1 bis 4, **dadurch gekennzeichnet, daß** sie ein zusätzliches physiologisch verträgliches Anion enthalten, und ihre physiologisch verträglichen Salze.

6. Wismutsalze gemäß einem oder mehreren der Ansprüche 1 bis 5, **dadurch gekennzeichnet, daß** sie das Wismut und das Antibiotikum oder die Antibiotika im Molverhältnis von ungefähr 1 : 1 enthalten, und ihre physiologisch verträglichen Salze.

7. Verfahren zur Herstellung von Wismutsalzen gemäß einem oder mehreren der Ansprüche 1 bis 6, **dadurch gekennzeichnet, daß** man das oder die Antibiotika der Moenomycin-Gruppe oder deren Salze in einem Lösungsmittel oder Dispergiermittel mit einem Wismutsalz umsetzt.

8. Wismutsalze gemäß einem oder mehreren der Ansprüche 1 bis 6 zur Anwendung als Arzneimittel.

9. Pharmazeutisches Präparat, enthaltend eine wirksame Menge mindestens eines Wismutsalzes gemäß einem oder mehreren der Ansprüche 1 bis 6 und/oder eines physiologisch verträglichen Salzes davon zusammen mit pharmazeutisch einwandfreien Trägerstoffen und/oder Hilfsstoffen.

10. Pharmazeutisches Präparat gemäß Anspruch 9, **dadurch gekennzeichnet, daß** es einen oder mehrere weitere Arzneimittelwirkstoffe zur Behandlung von Magenerkrankungen oder Ulcera enthält.

11. Verwendung eines Wismutsalzes gemäß einem oder mehreren der Ansprüche 1 bis 6 und/oder eines physiologisch verträglichen Salzes davon zur Herstellung eines Medikaments zur Therapie oder Prophylaxe von Ulcera, Magenerkrankungen, Magengeschwüren, Gastritis, oder zur Prophylaxe von Magenkrebs.

12. Verwendung eines Wismutsalzes gemäß einem oder mehreren der Ansprüche 1 bis 6 und/oder eines physiologisch verträglichen Salzes davon zur Herstellung eines Medikaments zur Bekämpfung von Helicobacter pylori.

## Claims

1. A bismuth salt of an antibiotic of the moenomycin group present individually or as a mixture, or its physiologically tolerable salts.

2. A bismuth salt as claimed in claim 1, which is derived from one or more of the antibiotics moenomycin, prasinomycin, diumycin, 11837 R.P., 8036 R.P., 19402 R.P., ensachomycin, prenomycin, teichomycin and pholipomycin, or its physiologically tolerable salts.

3. A bismuth salt as claimed in claims 1 and/or 2, which is derived from the moenomycin itself present in the form of an individual component or in the form of a mixture of individual components, or its physiologically tolerable salts.

4. A bismuth salt as claimed in one or more of claims 1 to 3, which is derived from moenomycin A and/or moenomycin C₃, or its physiologically tolerable salts.

5. A bismuth salt as claimed in one more of claims 1 to 4, which contains an additional physiologically tolerable anion, or its physiologically tolerable salts.

6. A bismuth salt as claimed in one or more of claims 1 to 5, which contains the bismuth and the antibiotic or the antibiotics in a molar ratio of approximately 1 : 1, or its physiologically tolerable salts.

7. A process for the preparation of bismuth salts as claimed in one or more of claims 1 to 6, which comprises reacting the antibiotic(s) of the moenomycin group or its/their salts with a bismuth salt in a solvent or dispersant.

8. The bismuth salt as claimed in one or more of claims 1 to 6 for use as pharmaceutical.

9. A pharmaceutical preparation, comprising an efficacious amount of at least one bismuth salt as claimed in one or more of claims 1 to 6 and/or of a physiologically tolerable salt thereof together with pharmaceutically innocuous excipients and/or auxiliaries.

10. A pharmaceutical preparation as claimed in claim 9, which contains one or more further pharmaceutical active compounds for the treatment of gastric disorders or ulcers.

11. The use of a bismuth salt as claimed in one or more of claims 1 to 6 and/or of a physiologically tolerable salt thereof for the production of a medicament for the therapy or prophylaxis of ulcers, gastric disorders, gastric ulcers, gastritis, or for the prophylaxis of stomach cancer.

12. The use of a bismuth salt as claimed in one or more of claims 1 to 6 and/or of a physiologically tolerable salt thereof for the production of a medicament for the control of Helicobacter pylori.

## Revendications

1. Sels de bismuth d'antibiotiques du groupe de la moénomycine présents individuellement ou en mélange, et leurs sels physiologiquement acceptables.

2. Sels de bismuth selon la revendication 1, **caractérisés en ce qu'**ils dérivent d'un ou plusieurs des antibiotiques moénomycine, prasinomycine, diumycine, 11837 R.P., 8036 R.P., 19402 R.P., ensachomycine, prénomycine, teichomycine et pholipomycine, et leurs sels physiologiquement acceptables.

3. Sels de bismuth selon la revendication 1 et/ou 2, **caractérisés en ce qu'**ils dérivent de la moénomycine sous forme d'un composant individuel ou sous forme d'un mélange de composants individuels, et leurs sels physiologiquement acceptables.

4. Sels de bismuth selon une ou plusieurs des revendications 1 à 3, **caractérisés en ce qu'**ils dérivent de la moénomycine A et/ou de la moénomycine C₃, et leurs sels physiologiquement acceptables.

5. Sels de bismuth selon une ou plusieurs des revendications 1 à 4, **caractérisés en ce qu'**ils contiennent un anion physiologiquement acceptable supplémentaire, et leurs sels physiologiquement acceptables.

6. Sels de bismuth selon une ou plusieurs des revendications 1 à 5, **caractérisés en ce qu'**ils contiennent le bismuth et l'antibiotique ou les antibiotiques dans le rapport molaire d'environ 1:1, et leurs sels physiologiquement acceptables.

7. Procédé de préparation de sels de bismuth selon une ou plusieurs des revendications 1 à 6, **caractérisé en ce que** l'on fait réagir l'antibiotique ou les antibiotiques du groupe de la moénomycine ou leurs sels dans un solvant ou dispersant avec un sel de bismuth.

8. Sels de bismuth selon une ou plusieurs des revendications 1 à 6 destinés à être utilisés comme médicaments.

9. Préparation pharmaceutique contenant une quantité efficace d'au moins un sel de bismuth selon une ou plusieurs des revendications 1 à 6 et/ou d'un sel physiologiquement acceptable de ceux-ci ainsi que des supports et/ou adjuvants pharmaceutiquement irréprochables.

10. Préparation pharmaceutique selon la revendication 9, **caractérisée en ce qu'**elle contient un ou plusieurs autres principes actifs médicamenteux pour le traitement des maladies gastriques ou des ulcères.

11. Utilisation d'un sel de bismuth selon une ou plusieurs des revendications 1 à 6 et/ou d'un sel physiologiquement acceptable de celui-ci pour la préparation d'un médicament pour la thérapie ou la prophylaxie des ulcères, des maladies gastriques, des ulcères gastriques, de la gastrite, ou pour la prophylaxie du cancer gastrique.

12. Utilisation d'un sel de bismuth selon une ou plusieurs des revendications 1 à 6 et/ou d'un sel physiologiquement acceptable de celui-ci pour la préparation d'un médicament pour lutter contre Helicobacter pylori.
